# EUROPEAN PATENT APPLICATION

(11) **EP 1 075 846 A2**
(43) Date of publication of application: **14.02.2001**
(21) Application number: 99917156.4
(22) Date of filing: 26.04.1999
(51) Int. Cl.: A61M 5/00

(54) **CUFF FOR BLOOD PRESSURE MANOMETER**

(30) Priority: 27.04.1998 JP 11692898
(71) Applicant: Holmes, James, Mississauge, Ontario L5A 3S2 (CA)
(72) Inventor: ITONAGA, Kazunobu Omron Corporation, Kyoto-shi Kyoto 616-8025 (JP); KATO, Hiroyuki Omron Corporation, Kyoto-shi Kyoto 616-8025 (JP); TANAKA, Takahide Omron Corporation, Kyoto-shi Kyoto 616-8025 (JP)
(74) Representative: Kilian, Helmut, Dr.
(86) International application number: JP9902208
(87) International publication number: WO9955400

(57) **Abstract**

A cuff 10 is constituted of a pressurization air bag 11 into which a predetermined amount of air is supplied to pressurize arteries 4 and 5 of a wrist portion 1, a press air bag 12 causing the pressurization air bag 11 to press the wrist portion 1, and a band 13 for attaching both air bags 11 and 12 to the wrist portion 1. When blood pressure is to be measured, a predetermined amount of air is supplied into the air bag 11 and then air is supplied into the air bag 12. The air bag 12 is accordingly expanded to cause the air bag 11 to pressurize the arteries 4 and 5. In this way, the cuff for sphygmomanometer is provided which can ensure blocking of blood flow in arteries so as to obtain measurements with higher precision and which can also eliminate discomfort caused by a feeling of being excessively pressurized, dampness and the like.

## Description

### Technical Field

The present invention relates to a cuff for a sphygmomanometer that is used by being attached to some region of a living body or subject for measuring blood pressure. In particular, the invention relates to a cuff most appropriate for a wrist sphygmomanometer for measuring blood pressure at the wrist portion.

### Background Art

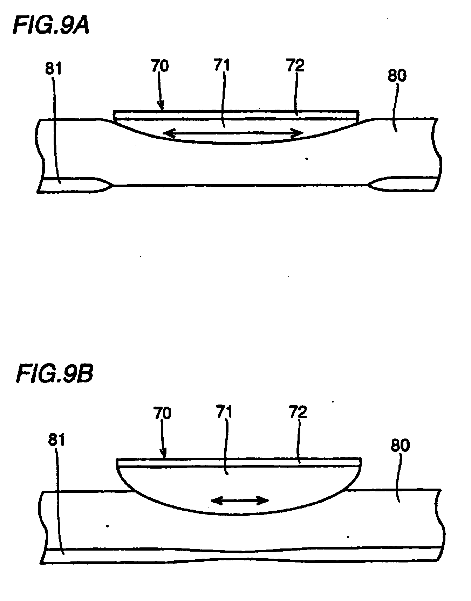

Figs. 9A and 9B illustrate a conventional sphygmomanometer cuff. As shown in Fig. 9, the conventional sphygmomanometer cuff 70 is constituted of an air bag 71 into which air is supplied and a strip-like band 72 for attaching air bag 71 to a certain region (arm, wrist) 80 of a living body or subject. Band 72 of cuff 70 is used to attach air bag 71 to subject region 80 and then air is supplied to air bag 71, so that an artery 81 of subject region 80 is pressurized and accordingly blood pressure is measured during the process of discharging air from air bag 71.

Japanese Patent Publication No. 6-28636 discloses "Sphygmomanometer Arm Band and Sphygmomanometer Pressurizing Device" as a prior art of the sphygmomanometer cuff. The sphygmomanometer arm band includes, in addition to a main body of the arm band, an inflatable bag and connecting means, an actuator for decreasing the diameter of the arm band by shortening the length of the arm band body. Regarding this arm band, the actuator is inflated before expansion of the inflatable bag and thus the arm band body is tightened up to an extent which is sufficient to measure blood pressure. The inflatable bag is thereafter distended to have a pressure sufficiently higher than the maximum blood pressure and then the air within the inflatable bag is gradually discharged. Blood pressure is thus measured during the discharging process.

In conventional cuff 70, the pressurizing force required to block the flow of blood in artery 81 is provided by the volume of expansion of air bag 71 only. If cuff 70 is closely attached to subject region 80 as shown in Fig. 9A, air bag 71 has a wide flat portion (effective range). When air bag 71 is distended in this state, the pressurizing force is conveyed to artery 81 which is enough to block the flow of blood in artery 81. On the other hand, if cuff 70 is attached loosely to subject region 80 as shown in Fig. 9B, air bag 71 has a relatively narrow effective range. Therefore, even if air bag 71 is inflated sufficiently in this state, the pressurizing force is not conveyed to artery 81 and accordingly the flow of blood in artery 81 is not blocked. In this case, an additional volume of expansion of air bag 71 is required corresponding to the space between air bag 71 and the skin, resulting in a measurement higher than a true value.

In order to avoid this problem, cuff 70 should be closely fit onto subject region 80 as shown in Fig. 9A. However, if cuff 70 stands being attached closely to subject region 80 all the time, there would arise a feeling of being excessively pressurized. Especially in the period of sweaty summer or rainy season, there would arise discomfort. The resultant problem is difficulty in constant attachment of the sphygmomanometer (particularly wrist sphygmomanometer) even if the sphygmomanometer body or cuff is reduced in size.

Regarding the arm band and pressurizing device disclosed in the patent above, the actuator operates to reduce the diameter of the arm band and the arm band is then closely attached to the subject in a manner similar to the conventional wrapping state, and the operation of the actuator is cancelled to allow the diameter of the arm band to increase and thus loosen the arm band. This approach advantageously achieves the effect of avoiding the discomfort caused by the feeling of being excessively pressurized and unpleasant dampness. However, in measurement of blood pressure, the actuator is first expanded to pull and accordingly tighten the arm band body and then the inflatable bag is distended until a predetermined pressure is obtained. Therefore, the inflatable bag is likely to be shifted from its original position. In other words, the possibility of the shift of the inflatable bag is higher when the actuator operates to pull the arm band body after the arm band is loosely attached at first. In the event of the shift of the inflatable bag, a sufficient degree of pressurizing force cannot be conveyed to the artery, resulting in the state shown in Fig. 9B. If the blood flow in the artery is to be blocked in this state, the volume of expansion of the inflatable bag must be increased, causing further discomfort such as the feeling of being excessively pressurized.

The present invention is made to address these problems and aims to provide a sphygmomanometer cuff which can ensure blocking of the blood flow in an artery so as to obtain measurements with higher precision and which can also eliminate discomfort caused by the feeling of being excessively pressurized, dampness and the like.

### Disclosure of the Invention

A sphygmomanometer cuff according to the present invention includes a fluid bag for pressurization into or in which a predetermined amount of fluid is supplied or confined to pressurize an artery of a region of a living subject, a press portion causing the pressurization fluid bag to press the subject region, and an attachment unit for attaching the pressurization fluid bag and the press portion to the subject region.

The pressurization fluid bag of the cuff is different from the conventional cuff and the inflatable bag disclosed in the patent above in that only a predetermined amount of fluid is supplied into the fluid bag or a predetermined amount of fluid is confined therein in advance and then the press portion causes the pressurization fluid bag to press the subject region. In measurement of blood pressure, the press portion may just be used to press the pressurization fluid bag against the subject region. Therefore, the cuff may be attached loosely to the subject region and accordingly the discomfort caused by the feeling of excess pressure and dampness can be avoided so as to enable the cuff to be attached all the time. Further, only a predetermined amount of fluid is supplied into or confined in the pressurization fluid bag, and the force for sufficiently pressurizing the subject region by the pressurization fluid bag is obtained from the press portion. Specifically, the press portion causes the pressurization fluid bag to press the subject region. By positioning the pressurization fluid bag at a predetermined site (where artery is pressurized) when the cuff is attached, even if the cuff is attached loosely, it is possible to prevent shift of the position of the pressurization fluid bag due to the operation of the press portion which hinders conveyance of a sufficient pressure to the artery. Measurements with higher precision can thus be obtained.

### Brief Description of the Drawings

Fig. 1 is a cross sectional view illustrating a main portion of a cuff attached to the wrist portion according to one embodiment of the invention.
Fig. 2 is a cross sectional view illustrating a main portion of a cuff attached to the wrist portion according to another embodiment of the invention.
Fig. 3 is a cross sectional view illustrating a main portion of a cuff attached to the wrist portion according to still another embodiment of the invention.
Fig. 4 is a cross sectional view illustrating a main portion of a modification of the cuff
Fig. 5 is a cross sectional view illustrating a main portion of a cuff attached to the wrist portion according to a further embodiment of the invention.
Fig. 6 is a block diagram illustrating a structure of a fluid system in a sphygmomanometer having any of the cuffs shown in Figs. 1 to 4.
Figs. 7A to 7C generally illustrate a structure and function of a three-way cock employed in the fluid system in Fig. 6.
Fig. 8 is a flow chart illustrating an overall operation of a sphygmomanometer having the fluid system in Fig. 6 with the three-way cock in Figs. 7A to 7C.
Fig. 9A illustrates a function of a conventional cuff which is attached closely to a subject region and Fig. 9B is a cross sectional view illustrating a function thereof which is attached loosely to the subject region.

### Best Modes for Carrying Out the Invention

The present invention is hereinafter described in conjunction with the embodiments thereof.

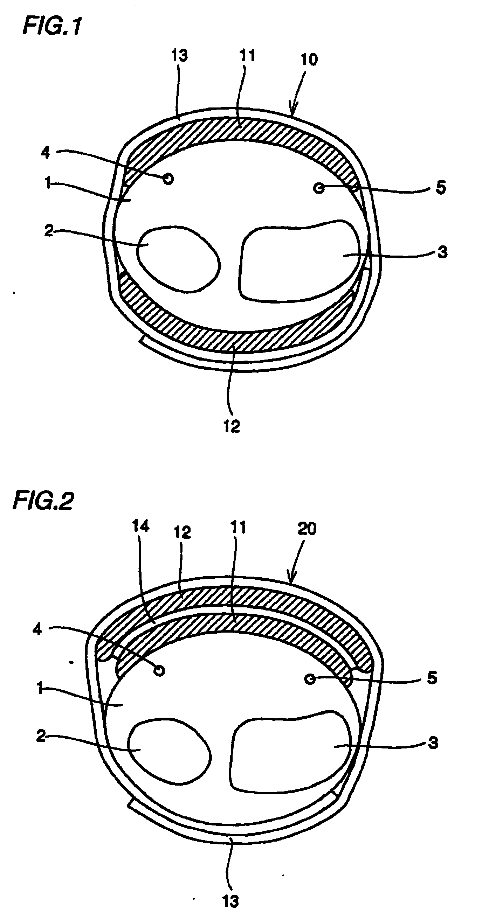

Referring to Fig. 1, a cuff 10 used for a wrist sphygmomanometer is constituted of an air bag for pressurization (pressurization fluid bag) 11 into which a predetermined amount of air is supplied so as to pressurize arteries 4 and 5 of a subject region (wrist portion) 1, an air bag for press (press fluid bag) 12 serving as a pressing section to cause pressurization air bag 11 to press wrist portion 1, and a strip-like band 13 serving as an attachment unit for attaching both air bags 11 and 12 to wrist portion 1. Pressurization air bag 11 and press air bag 12 of cuff 10 have respective widths almost equal to the transverse width of wrist portion 1. The bags are located opposite to each other with wrist portion 1 therebetween, and cuff 10 is attached to wrist portion 1 such that pressurization air bag 11 faces the inside of wrist portion 1. Band 13 has a hook-and-loop fastener so as to fix the wrapping state at an arbitrary position.

Cuff 10 is attached to wrist portion 1 as shown in Fig. 1. Specifically, cuff 10 is wrapped around wrist portion 1 by band 13 such that pressurization air bag 11 faces an ulnar artery 4 and a radial artery 5 while press air bag 12 faces the opposite side (an ulna 2 and a radius 3). It is noted that ulna 2 and ulnar artery 4 axe located on the little finger side of the hand and radius 3 and radial artery 5 are located on the thumb side in human wrist portion 1. It is unnecessary to tightly wrap cuff 10 around wrist portion 1 and the cuff may be wrapped loosely to an extent which is enough to prevent pressurization air bag 11 from shifting from the position facing arteries 4 and 5.

When blood pressure is to be measured, pressurization air bag 11 is first supplied with a predetermined amount of air and accordingly inflated. The amount of air to be supplied is not specified uniquely, however, the amount thereof should be enough to expand air bag 11 over the entire surface maintaining substantially flat shape when air is supplied before attachment of the cuff to the wrist portion.

Next, press air bag 12 is supplied with air and is thus inflated, causing pressurization air bag 11 to press wrist portion 1 (arteries 4 and 5). The air is supplied into air bag 12 until a predetermined pressure (pressure higher than the maximum blood pressure) is achieved. When the expansion of air bag 12 causes the pressurization force (internal pressure) of air bag 11 to reach the predetermined pressure, supply of air into air bag 12 is stopped. After this, the air within air bag 12 is gradually discharged and the blood pressure is measured during the process of discharge. After the measurement of blood pressure is completed, the air in air bag 11 is also discharged.

Pressurization air bag 11 of cuff 10 above is supplied with only a predetermined amount of air and accordingly air bag 11 does not exceed a predetermined degree of expansion. Air bag 11 is then pressed inwardly toward wrist portion 1 by the expansion of air bag 12. Consequently, even if cuff 10 is attached to wrist portion 1 by band 13 to meet the tightness according to the user's preference, air bag 11 is never shifted from its original position and the width of air bag 11 does not change. Therefore, the flow of blood in arteries 4 and 5 (especially radial artery 5) is blocked surely by air bag 11 and the resultant measurement of the blood pressure does not exceed a real value, achieving a measurement with higher precision. In other words, cuff 10 may be attached all the time by loosely wrapping it like a wrist watch, re-wrapping of cuff 10 to closely fit it onto the wrist for each measurement of blood pressure is unnecessary and thus the loose-fit state may be maintained to make measurement. Further, cuff 10 may be loosely wrapped when attached, eliminating discomfort such as the feeling of being excessively pressurized, unpleasant dampness and the like.

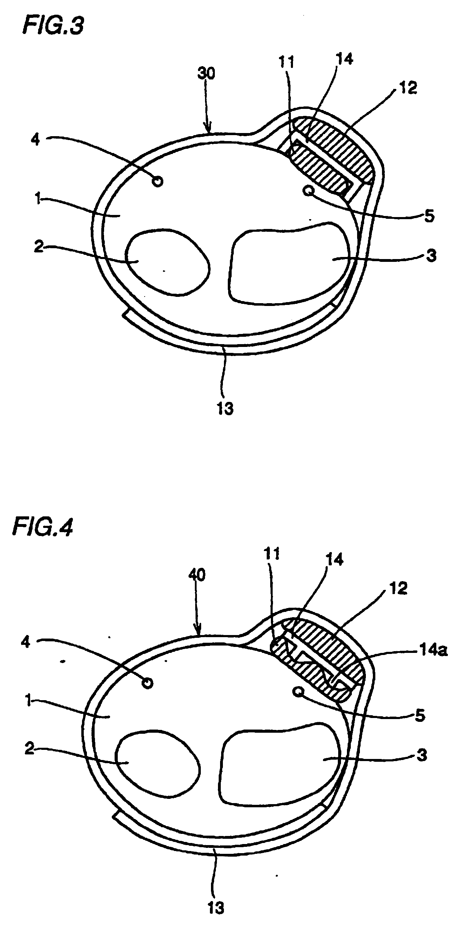

It is noted that the respective widths of pressurization air bag 11 and press air bag 12 (widths in the transverse direction of wrist portion 1) are almost equal to the width of wrist portion 1, however, especially air bag 11 may have a smaller width as shown in Figs. 3 and 4 which is enough to pressurize only radial artery 5. Further, the method of supplying a predetermined amount of air into air bag 11 may appropriately be selected from a method of supplying air by a manual pump a certain number of times, a method of supplying air by an electric pump at a certain voltage or for a certain period of time, a method of confining a certain amount of air in advance, and the like. In addition, measurement of the blood pressure maybe started any time which may be the start of the supply of a predetermined amount of air to air bag 11, during the period of the supply, after the supply and the like.

Fig. 2 is across sectional view of a main portion of a sphygmomanometer cuff which is attached to the wrist portion according to another embodiment. The components of the another embodiment corresponding to those of the one embodiment are denoted by the same reference characters. Cuff 20 has a press portion for causing a pressurization air bag 11 to press wrist portion 1, the press portion consisting of an intervening member 14 placed on the outside of pressurization air bag 11, and a press air bag 12 placed on the outside of intervening member 14. Intervening member 14 holds air bag 11 while it allows the pressure in air bag 12 to be exerted uniformly on air bag 11 and has a width almost equal to the width of air bag 11. It is noted that intervening member 14 may be formed of a sheet material like a curler (preformed plate) having an appropriate flexibility, rubber or sponge, or inflexible materials.

Press air bag 12 of cuff 20 is inflated and the pressurizing force thereof is conveyed via intervening member 14 to pressurization air bag 11, so that pressurization air bag 11 pressurizes arteries 4 and 5 to achieve the effects discussed above.

A cuff according to still another embodiment is shown in Fig. 3. A pressurization air bag 11 of cuff 30 has a minimum size (width) required for pressurizing only a radial artery 5. Pressurization air bag 11 is housed in an intervening member 14 and a press air bag 12 has a minimum size required to press the back of intervening member 14.

Cuff 30 is loosely wrapped around a wrist portion 1 such that pressurization air bag 11 faces radial artery 5 upon attachment. When blood pressure is to be measured, a predetermined amount of air is supplied into air bag 11 and thereafter air is supplied into air bag 12 so as to inflate air bag 12 and thus cause air bag 11 to pressurize artery 5. Since air bag 11 is positioned by intervening member 14 relative to artery 5, air bag 11 is never shifted from its original position even if the size thereof is small.

Fig. 4 illustrates a modification of cuff 30 in Fig. 3. A cuff 40 in Fig. 4 includes an intervening member 14 which has a leg portion 14a. Two linear leg portions 14a may be placed in parallel, or annular leg portion 14a may be placed. Leg portion 14a of intervening member 14 of cuff 40 surely positions air bag 11 relative to artery 5.

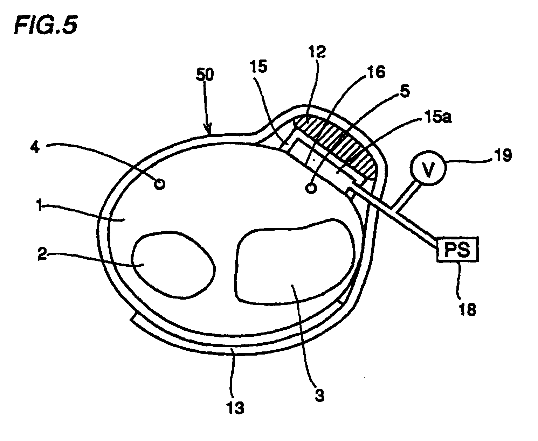

A cuff according to a further embodiment is shown in Fig. 5. A pressurization fluid bag of cuff 50 is formed of an air introduction member (fluid introduction member) 15 having a space 15a into which air is supplied, and a press film 16 attached to air introduction member 15 to seal space 15a. Space 15a of air introduction member 15 is connected to a pressure sensor 18 through a flow path (e.g. tube), and an open-to-air valve 19 is provided on the way through the flow path.

When blood pressure is not measured, valve 19 of cuff 50 is opened so as to open space 15a of air introduction member 15. When measurement of the blood pressure is to be done, valve 19 is closed and the internal pressure of space 15a is detected by pressure sensor 18 while air is supplied into space 15a until the internal pressure reaches a predetermined pressure. Then, press film 16 bulges to moderately press a wrist portion 1. Air is thereafter supplied into an air bag 12 to inflate air bag 12 so as to press air introduction member 15 by the inflation of air bag 12 and accordingly cause press film 16 to pressurize an artery 5.

The following is another operation manner. When blood pressure is to be measured, valve 19 is dosed and the internal pressure of space 15a is detected by pressure sensor 18. With a predetermined amount of air confined in space 15a, air may be supplied into air bag 12 to press air introduction member 15 by expansion of air bag 12 and accordingly cause press film 16 to pressurize artery 5.

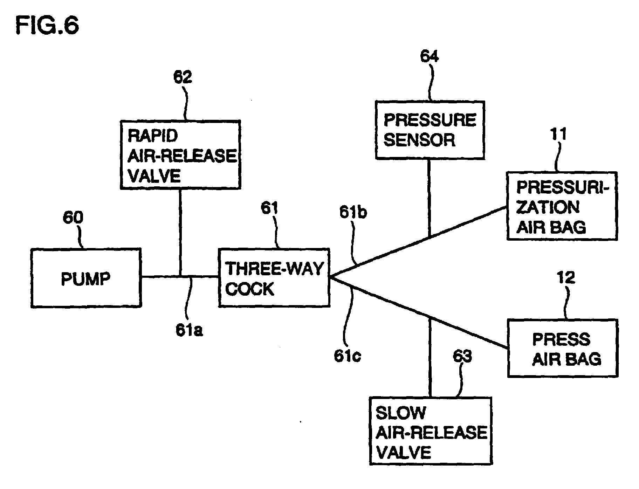

Fig. 6 is a block diagram illustrating a structure of a fluid system associated with pressurization air bag 11 and press air bag 12 in any of cuffs 10, 20, 30 and 40 illustrated respectively in Figs. 1 to 4. In this fluid system structure, pressurization air bag 11 and press air bag 12 are connected to a pump 60 of an air supply source via a three-way cock 61. Further, a rapid air-release valve 62 is connected to a flow path 61a between pump 60 and three-way cock 61, a slow air-release valve 63 is connected to a flow path 61c between three-way cock 61 and press air bag 12, and a pressure sensor 64 is connected to a flow path 61b between three-way cock 61 and pressurization air bag 11.

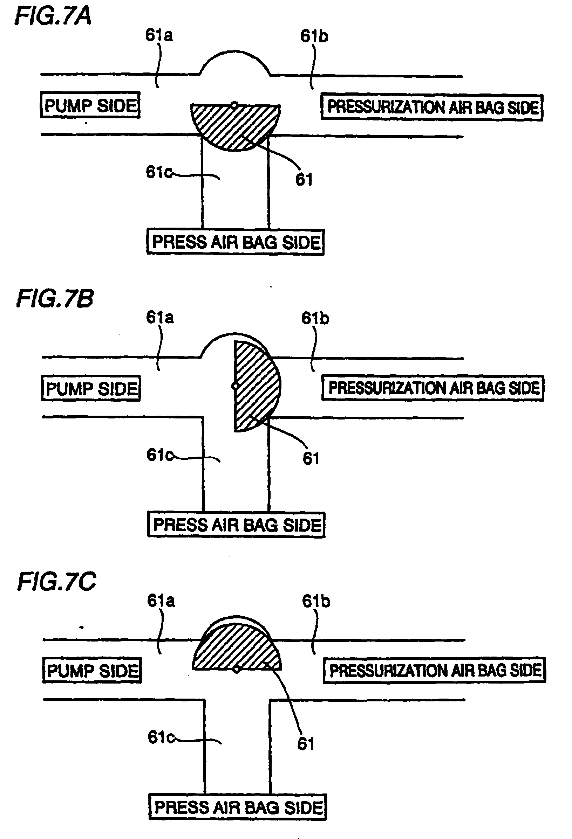

Three-way cock 61 having the structure and function generally illustrated in Figs. 7A to 7C establishes communication between two or all of the three flow paths, i.e. flow path 61a associated with pump 60, flow path 61b associated with pressurization air bag 11, and flow path 61c associated with press air bag 12. When there is established communication between flow paths 61a and 61b and flow path 61c is closed as shown in Fig. 7A, air is supplied from pump 60 to pressurization air bag 11 only. When communication is established between flow paths 61a and 61c and flow path 61b is closed as shown in Fig. 7B, air from pump 60 is supplied to press air bag 12 only. When communication is established between three flow paths 61a, 61b and 61c as shown in Fig. 7C, air from pump 60 is supplied to both of pressurization air bag 11 and press air bag 12.

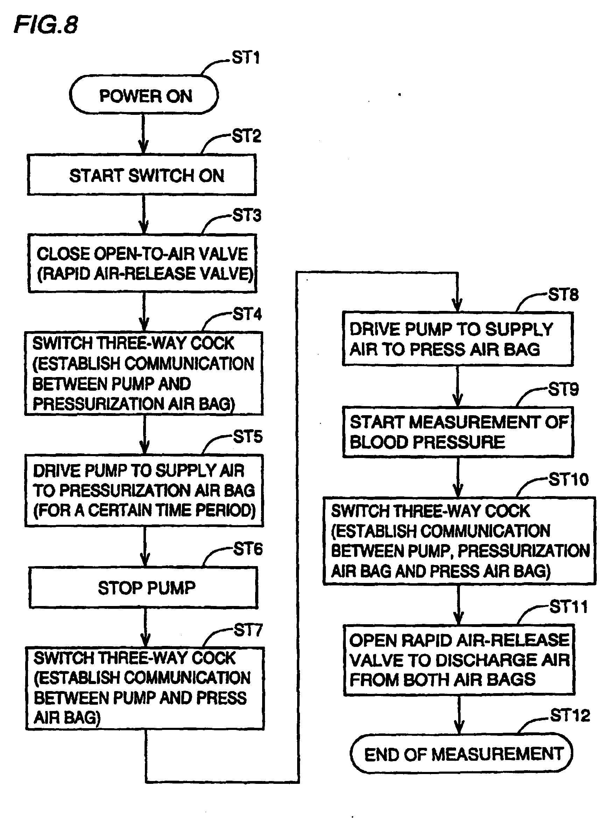

Referring to the flow chart shown in Fig. 8, an overall operation of a sphygmomanometer (wrist sphygmomanometer) having the fluid system in Fig. 6 with three-way cock 61 of the structure as shown in Figs. 7A to 7C is now described. The power of the sphygmomanometer is first turned on (step 1, hereinafter abbreviated as ST1), then a start switch is turned on (ST2), and rapid air-release valve (open-to-air valve) 62 is accordingly dosed (ST3). Next, three-way cock 61 switches to the state in Fig. 7A to allow pump 60 and pressurization air bag 11 to communicate with each other (ST4).

Next, pump 60 operates to supply air into pressurization air bag 11 for a certain period of time (by a predetermined amount) (ST5). After a certain time period has passed, pump 60 is stopped (ST6), and accordingly three-way cock 61 switches to the state in Fig. 7B to allow pump 60 and press air bag 12 to communicate with each other (ST7). Then, pump 60 operates to supply air into press air bag 12 (ST8). Air bag 12 is thus expanded, causing air bag 11 to pressurize a measurement site (wrist portion) sufficiently to start measurement of blood pressure (ST9). At this time, slow air-release valve 63 opens to gradually discharge air from press air bag 12.

When the measurement of blood pressure is completed, three-way cock 61 switches to the position shown in Fig. 7C, so that communication is established between pump 60, pressurization air bag 11 and press air bag 12 (ST10). Rapid air-release valve 62 is then opened to discharge air from both air bags 11 and 12 (ST11). Completion of the air release ends the measurement of blood pressure (ST12).

The embodiments above are all applied to the cuffs for the wrist sphygmomanometers. However, they are similarly applicable to a cuff which is wrapped around the arm. I addition, although the press portions are all air bags for press, they may be implemented as a band which is wrapped around a subject region and has an automatic adjustment mechanism for circumferential length. The automatic adjustment mechanism for circumferential length is composed of a gear and a motor, for example, and the motor operates to rotate the gear for adjustment of the circumferential length of the band in order to lengthen or shorten it. When the circumferential length of the band is shortened, pressurization air bag 11 (Figs. 1 to 4) or press film 16 (Fig. 5) pressurizes the artery. If the band having the automatic circumferential adjustment mechanism is employed, this band also serves as band 13 as the attachment unit.

Although the air bag, to and from which air is supplied and discharged, is employed as a fluid bag, the air bag may be any fluid bag to and from which any fluid except the air (gas such as oxygen and carbon dioxide or liquid such as water) is supplied and discharged. In particular, if hydrogen liquid is used, it is necessary to construct a liquid-tight fluid system to prevent leakage of the liquid..

### Industrial Applicability

As heretofore discussed, the sphygmomanometer cuff according to the present invention has the pressurization fluid bag which may be caused to pressurize a subject region by the press portion for measurement of blood pressure. The cuff may accordingly be attached loosely to the subject region, which eliminates discomfort caused by feeling of being excessively pressurized, dampness and the like. For this reason, the cuff is employed in blood pressure application as the one that can be kept attached.

## Claims

1. A sphygmomanometer cuff comprising a pressurization fluid bag into or in which a predetermined amount of fluid is supplied or confined to pressurize an artery of a subject region, a press portion causing said pressurization fluid bag to press the subject region, and an attachment unit attaching said pressurization fluid bag and said press portion to the subject region.

2. The sphygmomanometer cuff according to claim 1, wherein
said press portion is a fluid bag for press placed opposite to said pressurization fluid bag with the subject region therebetween.

3. The sphygmomanometer cuff according to claim 1, wherein
said press portion is formed of an intervening member placed outside said pressurization fluid bag and a fluid bag for press placed outside said intervening member.

4. The sphygmomanometer cuff according to claim 1, wherein
said press portion is a band wrapped around the subject region and having an automatic circumferential adjustment mechanism.

5. The sphygmomanometer cuff according to claim 3, wherein
said pressurization fluid bag is positioned relative to an artery by said intervening member.

6. The sphygmomanometer cuff according to any of claims 1-5, wherein
said pressurization fluid bag is formed of a fluid introduction member having a space into which fluid is supplied and a press film attached to said fluid introduction member so as to seal the space.
